# EUROPÄISCHE PATENTSCHRIFT

(11) **EP 0 460 588 B1**
(45) Veröffentlichungstag und Bekanntmachung des Hinweises auf die Patenterteilung: **30.08.1995**
(21) Anmeldenummer: 91109076.9
(22) Anmeldetag: 04.06.1991
(51) Int. Cl.: A61K 9/70, A61K 9/20

(54) **Herstellungsverfahren für schnellzerfallende folienförmige Darreichungsformen**
Manufacture process of fast-desintegrating film-formed administration forms
Procédé d'obtention de formes d'administration en forme de feuille et à désagrégation rapide

(30) Priorität: 07.06.1990 DE 4018247
(43) Veröffentlichungstag der Anmeldung: 11.12.1991
(73) Patentinhaber: LTS LOHMANN THERAPIE-SYSTEME GmbH & CO.KG, 56513 Neuwied (DE)
(72) Erfinder: Horstmann, Michael, Dr., W-5450 Neuwied 1 (DE); Laux, Wolfgang, W-6252 Dietz (DE); Hungerbach, Stefan, W-5401 Nörtershausen (DE)
(74) Vertreter: Flaccus, Rolf-Dieter, Dr.

(56) Entgegenhaltungen:
- EP-A- 0 259 749
- DE-A- 3 744 009
- FR-A- 2 571 253
- GB-A- 2 009 597
- US-A- 4 136 145

## Beschreibung

Die Erfindung betrifft folienförmige Darreichungsformen von Arzneimitteln, Süßwaren, anderen Lebensmitteln, Kosmetika und dergleichen zur oralen Anwendung oder Einnahme.

Im Mundbereich anzuwendende oder durch Verschlucken einzunehmende Darreichungsformen von Arzneimitteln, Süßwaren und anderen Lebensmitteln sowie Kosmetika können grundsätzlich entweder vom Anwender frei portioniert werden oder sie sind bereits vom Hersteller in Dosiseinheiten vorzerteilt.
Solche vorportionierten Formen sind besonders im Arzneimittelbereich in Form von Tabletten, Kapseln, Dragees, Pillen, Trinkampullen und dergleichen sehr verbreitet.
Anders als zum Beispiel bei selbst zu dosierenden Formen wie Tropflösungen, Salben oder Cremes wird hier die unabsichtliche Fehldosierung durch den Anwender vermieden.

Dabei muß aus anwendungstechnischen Gründen bei Tabletten eine bestimmte Mindestgröße (5 bis 6 mm Durchmesser) und ein bestimmtes Mindestgewicht (ca. 100 bis 200 mg) eingehalten werden, damit diese Darreichungsformen auch handhabbar sind. So kann bei hochwirksamen Arzneistoffen der Hilfsstoffanteil rund 99% des Tablettengewichtes ausmachen.

Sogar Lebensmittel, vor allem Süßwaren, finden sich heute häufig in einzeln dosierter Form (Bonbons, Süßstofftabletten, Pfefferminzpastillen etc.) im Handel. Auch hier drängt sich - zum Beispiel bei Bonbons - der Wunsch auf, mit weniger "Hilfsstoff" (hier i.d.R. Zucker) das erwünschte Geschmackserlebnis zu erzielen.

Aus diesem Grund gibt es in letzter Zeit verstärkt technische Lösungsvorschläge mit dem Ziel, Darreichungsformen mit weniger Hilfsstoffmenge auszustatten und gleichzeitig einen sicheren manuellen Umgang mit diesen Mitteln zu gewährleisten.

Eine besonders interessante Möglichkeit hierzu ist die Herstellung folienförmiger Wirkstoffträger, wie sie zum Beispiel in DE 35 34 983, DE 27 46 414, BE 637 363, DE 24 32 925 oder DE 36 30 603 vorgeschlagen sind.

Nach den hierzu bekannten Herstellungsverfahren lassen sich zum einen solche papierähnlichen Träger zunächst wirkstofffrei herstellen und dann durch Aufsprühen (z.B. GB 1 061 557) einer wirkstoffhaltigen Lösung oder Überschichten bzw. Bedrucken mit einer konzentrierteren wirkstoffhaltigen Schicht (z.B. EP 0 219 762) erzeugen.

Für den Fachmann dürfte allerdings in erster Linie das gemeinsame Auf- oder Anlösen von Wirk- und Hilfsstoffen in Wasser oder anderen Lösungsmitteln (DE 24 49 865) mit anschließendem Ausstreichen - oder Ausgießen (z.B. JP 69026674) - und Trocknen das Verfahren der Wahl darstellen.
Auch die Extrusion unter Hitzeeinwirkung wurde bereits vorgeschlagen (Research Disclosure, 1986, No. 263, March, 145-146 (No. 26341)).

Folienförmige Darreichungsformen bieten auf unterschiedlichste Weise Möglichkeiten zur Unterteilung der Einzelrationen.
Man kann sie sich in Form von Briefmarkenbögen vorstellen, bei denen die Portion erst kurz vor Verwendung aus dem Gesamtverbund entnommen wird (z.B. BE 637 363, EP 0 219 762), es lassen sich auch auf einer bandförmigen Darreichungsform, z.B. durch Perforation unterschiedlich große Einzeldosen bereitstellen (z.B. DE 27 46 414). Besonders bei Verwendung eines nicht eßbaren Trägers (z.B. DE 36 30 603) bietet sich jedoch die vollständige Trennung in Einzelportionen an, da die Entnahme so hygienischer, komfortabler und sicherer möglich ist.

Während also bereits eine Reihe von flächenförmigen Darreichungsformen und Herstellungsverfahren dafür zum Stand der Technik gehört, besteht noch ein erheblicher Mangel beim galenischen Aufbau, also bei der Hilfsstoffauswahl und im Konzept der physikochemischen Feinstruktur solcher Folienträger.
Hier sind unterschiedlichste Zielsetzungen zu berücksichtigen: Von vielen Arzneimitteln verlangt man schnellen Zerfall, damit die Dosierungsform rasch verschluckt werden kann, von anderen wiederum ist ein zeitweiliger klebender Aufenthalt auf der Mundschleimhaut erwünscht und in anderen Fällen - zum Beispiel bei kurzwirksamen arzneilichen Wirkstoffen - ist ein stark verzögerter Zerfall erwünscht. Bei Süßwaren dürfte im allgemeinen eine mittlere Verweilzeit zum Beispiel von Aromen im Mund erstrebenswert sein, eine kosmetische folienförmige Zahnpasta sollte möglichst schnell zerfallen und flexibel sein. Generell dürfen folienartige Darreichungsformen nicht zu leicht brüchig sein, um die Portion auch sicher zum Bestimmungsort bringen zu können. Bei Verwendung eines Trägers müssen beide Materialien ein genau angepasstes Adhäsionsverhalten zueinander aufweisen, um einerseits herstellungstechnisch die Vereinzelung der Dosen zum Beispiel durch teilweises Einstanzen und Abziehen der Stege sicher zu gewährleisten und andererseits die Anbindung an den Träger auch während der Lagerung zu sichern. Sofern Trocknung bei der Herstellung erforderlich ist, sollte die Rezeptur mit einem möglichst kleinen Lösemittelanteil (vorzugsweise Wasser) in einen streichfähigen Zustand zu bringen sein, damit nur wenig Trocknungsenergie aufgewandt werden muß.

Bisher bekannt gewordene galenische Konzepte werden diesen Anforderungen nur lückenhaft gerecht. Grundprinzipien der Tablettentechnologie werden in DE 27 46 414 im Zusammenhang mit folienartigen Bändern beschrieben, wie die Verwendung von optional thermoplastischen Bindemitteln und anderen Hilfsstoffen, die chemische Vernetzung oder der Zusatz hydrophober Stoffe zur Zerfallsverzögerung, die Vereinigung mehrerer Schichten und die Verwendung mikroverkapselter Wirkstoffe. Gewöhnliche Tablettensprengmittel wurden dort als Zerfallshilfsmittel für Folien angegeben. Nach eigenen experimentellen Erkenntnissen werden diese Vorschläge der neuen Arzneiform nicht gerecht. Klassische Sprengmittel erfordern eine poröse, durch interpartikuläre Bindungskräfte mechanisch stabile Umgebung, wenn sie bei Wasserzusatz durch Quellung oder gespeicherte Hookesche Verformungsenergie eine Desintegration bewirken sollen.
In den stets leicht flexibel bleibenden, nur wenig porösen folienförmigen Darreichungsformen sind diese Voraussetzungen jedoch nicht gegeben. Quellende Partikel können sogar durch Wasserentzug den Zerfall der Folien noch verzögern.

Nach der DE 24 32 925 enthält eine Rezeptur wasserlösliche Celluloseether und Trennmittel sowie gegebenenfalls Füllstoffe.
Da ein überwiegender Anteil von wasserlöslichen Polymeren jedoch regelmäßig hohen Wasserzusatz erfordert, um genügend niedrige Viskosität der streich- oder gießfertigen Masse zu erzielen, hat dieser Aufbau hohe Trocknungskosten in der Fertigung zur Folge.

Auch wird bei so starker Materialschrumpfung die adhäsive Bindung an den Untergrund belastet.
Beim Streichen von Massen auf Wasserbasis bei der Herstellung folienförmiger Applikationsformen auf ein dehäsiv ausgerüstetes Papier oder eine dehäsiv ausgerüstete Folie perlt die Flüssigkeit leicht vom Untergrund ab oder bildet zumindest aufgrund der Oberflächenspannung Zonen unterschiedlicher Filmdicke aus. Die Viskosität kann zwar durch Zusätze von Cellulosederivaten etc. erhöht werden, aber dann ist das Ausstreichen durch den dünnen Spalt nicht mehr problemlos möglich.
Nach anderer Quelle, z.B. DE 35 34 981, DE 36 30 603, werden daher Viskositätsbildner bevorzugt, welche in der Hitze in der Streichvorrichtung niedrigviskose Lösungen ergeben, unmittelbar danach aber beim Abkühlen gelartig stabilisierte Filme liefern, die dann an der Luft fehlerfrei trocknen, wie z.B. Agar-Agar oder Gelatine. Diese Methode ist jedoch nicht zufriedenstellend, da sich bei der Trocknung keine hohen Temperaturen anwenden lassen, weil die Masse wieder abperlt. Eine Trocknung solch wasserreicher Massen bei niedrigen Temperaturen ist aber andererseits wegen der hohen Verweilzeiten in der Maschine unökonomisch.

Die gleiche Schwierigkeit ergibt sich bei einer Darreichungsform entsprechend einem in der US-A-4 136 145 beschriebenen Verfahren. Es handelt sich um die Herstellung einer Darreichungsform mit einem Film als Träger, in dem ein Wirkstoff homogen dispergiert ist. Die Auftragsmasse enthält bis 60 Gew.-% Medikament, 0-30 % pharmazeutisch akzeptablen Füller, eine filmbildende Menge wasserlösliches Polymer sowie Lösungsmittel oder Suspensionsmedium Wasser bzw. organisches, flüchtiges, polares Lösungsmittel. Nach Beschichtung des Films wird die Masse getrocknet. Diese kann entsprechend einer Ausgestaltung 6-20 Gew.-% filmbildendes Polymer, 48-84 Lösungs- oder Suspensionsmedium und 0,01-2 % pharmazeutischen Wirkstoff enthalten.

Aus der EP-A-0 259 749 ist eine weitere Darreichungs- und Dosierungsform für Arzneimittelwirkstoffe, Reagentien oder dergleichen sowie ein Verfahren zu deren Herstellung bekannt. Sie weist ein Trägermaterial in Form eines release-Papiers, eines release-Films oder einer release-Folie auf, die einseitig mit einer wirkstoffhaltigen Beschichtung versehen ist, welche nach Vorzerteilung in Dosiereinheiten von dem Trägermaterial abziehbar ist. Die abgezogenen wirkstoffhaltigen Abschnitte eignen sich als orale Arzneimittel. Zum Naßauftrag auf einem release-Papier wird eine Beschichtungsmasse von schleimartiger Konsistenz hergestellt, sodann mittels eines Walzenüabertragungsverfahrens auf den Träger aufgebracht und getrocknet. Die Rezeptur der Beschichtungsmasse wird von Fall zu Fall dem darin enthaltenen Wirkstoff angepaßt.

Aus der FR-A-2 571 253 ist weiterhin die Herstellung filmförmiger Verabreichungsformen bekannt, welche ebenfalls eine aktive Komponente enthalten. Dabei wird von einer Mischung ausgegangen, welche Gelatine, Gluten, Carpoxyvinylpolymer, gomme guar, Lanolin enthält und mit einer größeren Menge Wasser vermischt wird, wonach in die schleimartige Masse ein oder mehrere Wirkstoffe eingearbeitet werden und diese dann bis zu einem Wasser-Restgehalt von 10 % getrocknet und auf einen Träger aufgebracht wird.

Der Erfindung lag daher die Aufgabe zugrunde, eine schnell in Wasser zerfallende einzeln dosierte folienförmige Darreichungsform sowie ein Verfahren zu ihrer Herstellung bereitzustellen, welches zur Verarbeitung nur einen sehr geringen Wasserzusatz erfordert, um eine ausreichende Dünnflüssigkeit zur Dosierung über Rakel- oder Walzenauftrag zu erreichen, dabei aber einen einheitlich dicken Film auf einem dehäsiven Träger ergibt, sowie im trockenen Zustand einerseits gut am Träger haftet, sich aber andererseits auch bei de Weiterverarbeitung, z.B. dem Vereinzeln durch Stanzen oder vor der Anwendung mit hinreichender Leichtigkeit von diesem ablösen läßt.
Diese Aufgabe wird erfindungsgemäß gelöst durch eine Darreichungsform, deren Zusammensetzung gemäß dem im Hauptanspruch gekennzeichneten Herstellungsprozeß aus den dazu vorgesehenen Substanzen gewonnen wurde.
Weitere Ausgestaltungen der Darreichungsformen sind entsprechend den Unteransprüchen vorgesehen.
Die erfindungsgemäßen Formulierungen lassen sich überraschenderweise bereits mit geringem Zusatz eines polaren Lösungsmittels in einen streichfähigen Zustand bringen.
Anders als Formulierungen nach dem Stand der Technik ergeben sie dabei bereits im kalten Zustand auf dehäsiv ausgerüsteten Trägern gleichmäßige Filme. Die Erzeugnisse können im getrockneten Zustand durch Stanzung so vereinzelt werden, daß die Einzeldosen auf einen gemeinsamen Träger des zum Streichen und Trocknen verwendeten Trägers verbleiben.
Die erfindungsgemäße Darreichungsform zerfällt im Mund innerhalb von 10 Minuten vollständig und kann ausschließlich aus Bestandteilen hergestellt werden, die nach dem geltenden Lebensmittelrecht der Bundesrepublik Deutschland zulässig sind. Als Füllstoffe sind z.B. Carbonate, Phosphat, Silicate, Sulfate oder Oxide der Erdalkalimetalle, Zinkoxid Siliciumoxide, Cellulose und ihre Derivate, Talkum oder Titandioxid verwendbar, aber auch schwerlösliche Zucker bzw. Zuckerderivate, wie zum Beispiel Lactose, oder Stärkederivate wie Cyclodextrine, sofern diese im Produkt im wesentlichen ungelöst vorliegen und damit die mechnischen Eigenschaften eines Füllstoffes erfüllen.

Unter dem Begriff Filmbildner werden solche Ingredienzien wie Zucker, Zuckeralkohole und ihre Derivate wie Rohrzucker, Sorbit, Mannit, Xylit, Glucose, Fructose, Lactose, Galactose verstanden, niedermolekulare organische Säuren wie Bernsteinsäure, Äpfelsäure oder Adipinsäure, Polyethylenglykol oder Mischungen solcher Substanzen wie zum Beispiel Honig.

Als dritte wesentliche erfindungsgemäße Komponente ist ein in Wasser quellbarer Gelbildner notwendig, der in der Regel auf der Basis polymerer Kohlenhydrate aufgebaut ist, wie zum Beispiel Stärke und ihre Derivate, Agar-Agar, Alginsäure, Arabinogalactan, Galactomannan, Cellulose und ihre Derivate, Carrageen, Dextran, Traganth und viele Gummen pflanzlicher Herkunft. Aber auch synthetische Polymere, die in Wasser löslich oder quellbar sind, lassen sich erfindungsgemäß einsetzen: Polyvinylpyrrolidon, Polyvinylalkohol, Polyacrylsäure oder Polyacrylamid, um nur einige zu nennen. Selbst Polypeptide wie Gelatine, Albumin, Kollagen oder Eiklar lassen sich verwenden. Vorteilhaft können Gemische von Gelbilduern aus polymeren Kohlenhydraten oder deren Derivaten, Gelatine, Carboxyvinylcopolymeren oder Polyvinylalkohol verwendet werden.

Eine als Füllstoff wirkende Komponente, Filmbildner und Gelbildner sind notwendige Bestandteile, die dem Produkt die gewünschten Eigenschaften regelmäßig nur dann verleihen, wenn sie bei Festlegung der quantitativen Rezeptur nach den jeweiligen Substanzeigenschaften gemeinsam verarbeitet worden sind. Dabei kann ein Füllstoff, aber auch ein gleichzeitig als Füllstoff wirkender Wirkstoff eingesetzt werden.
Die genannten vorteilhaften Eigenschaften treten bei Einhaltung bestimmter Intervalle von Mischungsverhältnissen auf:
An Filmbildner 20 bis 60 Gew.-%, an Gelbildner 2 bis 40 Gew.-%, an Wirkstoff 0,1 bis 35 Gew.-% und an Füllstoff bis 40 Gew.-%.

Die Verarbeitung kann nach den dem Fachmann bekannten Verfahren geschehen.
In der Regel wird man die Ausgangskomponenten trocken vormischen und dann unter Zusatz von maximal 30 Gew.-% eines polaren Lösemittels unter Rühren in eine streichfähige Konsistenz bringen. Die Verwendung von Homogenisatoren zur intensiveren Vermischung oder von Vakuum zur Entfernung von Luftblasen kann nützlich sein.

Bevorzugt werden Dispergier- und Mahleinrichtungen mit frei beweglichen Mahlkörpern (Kugelmühlen) an dieser Stelle eingesetzt.
Abhängig von den speziellen Eigenschaften der Gel- und Filmbildner kann die Anwendung von Hitze den Dispergierprozeß beschleunigen und die gewünschte physikochemische Beschaffenheit des Vorproduktes herbeiführen. Auch kann so unter Umständen der Wasserzusatz entbehrlich sein, wenn der Filmbildner schmilzt.
Es resultiert eine äußerlich homogen erscheinende, streich- oder extrudierbare Masse.
Die Formgebung geht im allgemeinen mit Streich-/Rakel- oder Extrusionsverfahren vonstatten, in denen die Masse einen Spalt definierter Dicke passiert, beispielsweise eine Schlitzdüse bei der Extrusion, und so die äußere Form erhält.
Ist noch Lösungsmittel enthalten, wird dies in geeigneten, dem Fachmann bekannten Trocknungseinrichtungen zumindest teilweise entzogen.

Vorteilhaft wird das Produkt auf einem Träger getrocknet, auf dem es auch noch nach der Trocknung durch Adhäsion haftet. Ist aus verfahrenstechnischen Gründen keine ausreichende Dicke des Vorproduktes zu erreichen, können zwei oder mehrere Schichten durch Anwendung von Druck und gegebenenfalls Wärme aufeinanderkaschiert werden.

Die Zerteilung in Einzeldosen geschieht durch Schneiden, Stanzen, Prägen und vergleichbare Verfahren, die flächenmäßig definierte, abgeteilte oder abteilbare Areale schaffen. Wird auf einem Träger getrocknet, kann die Darreichungsform nach diesem Vereinzelungsvorgang bis zur Anwendung auf dem Träger verbleiben, was die Entnahme sehr erleichtert.

Die Erfindung wird anhand der folgenden Beispiele veranschaulicht: 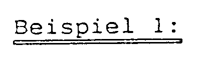
75 g acetylierte Stärke
62 g Honig
55 g Calciumsulfat-Dihydrat
5 g Citronensäure
50 g Wasser
werden in einer geschlossenen Rührapparatur gemischt und auf 50°C erhitzt. Die Masse wird noch zwei Stunden unter Rühren homogenisiert und anschließend auf Raumtemperatur abgekühlt. Unter Vakuum wird eine weitere halbe Stunde gerührt und das durch Verdampfen entzogene Wasser ersetzt.

2 ml Pfefferminzöl werden zugesetzt und innerhalb von 5 Minuten durch Rühren homogen eingearbeitet.

Die Masse wird in einer Spaltbreite von 500 Mikrometern mit einer Rakelvorrichtung auf siliconisiertes Papier gestrichen und 15 Minuten bei 80°C getrocknet.

Mit einer hierzu geeigneten Schneidvorrichtung werden die spätere Form konturierende Schnitte in die getrocknete Masse hinein geführt, ohne den Papierträger zu beschädigen.
Das zwischen den nun erhaltenen einzeln dosierten Darreichungsformen verbliebene Material wird in einem Arbeitsgang durch mechanisches Abziehen entfernt.
Jeweils 12 dieser Darreichungsformen werden zum Schutz vor Austrocknung auf einem gemeinsamen Stück Trägerpapier in einem im wesentlichen wasserdampfundurchlässigen Papier/Aluminium/Ethylenvinylacetat-Verbundpackstoff eingesiegelt.
- Verwendung:: Aromaträger (Süßwaren)

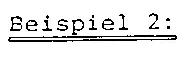

100 g Polyethylenglykol (Molekulargewicht ca. 1500 g/Mol)
8 g Carboxyvinylcopolymer
werden in einem heizbaren Doppel-Z-Kneter bei 80°C bis zur Homogenität verknetet (Dauer: 2 Stunden).
70 g Lactose
werden zugegeben und innerhalb von 30 Minuten in die Grundlage eingeknetet. Die Temperatur wid auf 50°C reduziert. Nun werden noch
8 g Glibenclamid
zugegeben und der Ansatz weitere 30 Minuten verknetet. Die heiße Mischung wird in einen auf 50°C vorgeheizten Kolbenextruder (Nutzvolumen ca. 150 ml) gefüllt. Sofort wird mit einer Fördergeschwindigkeit von ca. 10 g/min durch eine 10x1 mm große Flachdüse extrudiert und auf einer kalten, sauberen Arbeitsfläche bis zur Erstarrung abgekühlt. Der Strang wird in 10 mm breite Abschnitte durch Messerschnitt geteilt, so daß ca. 80 mg schwere, im Mund zerfallende orale Arzneiformen mit ca. 3 mg Wirkstoffgehalt entstehen. 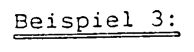
25 g acetylierte Stärke
20 g Sorbit
30 g Calciumcarbonat
1 g Titandioxid
22 g Wasser
8 g Glycerin
werden in einer geschlossenen Rührapparatur gemischt und auf 50°C erhitzt. Die Masse wird noch zwei Stunden unter Rühren homogenisiert und anschließend auf Raumtemperatur abgekühlt. Unter Vakuum wird eine weitere halbe Stunde gerührt und das durch Verdampfen entzogene Wasser ersetzt.
0,5 ml Pfefferminzöl werden zugesetzt und innerhalb von 5 Minuten durch Rühren homogen eingearbeitet.
Die Masse wird in einer Spaltbreite von 500 Mikrometern mit einer Rakelvorrichtung auf siliconisiertes Papier gestrichen und 10 Minuten bei 80°C getrocknet.

Mit einer hierzu geeigneten Schneidvorrichtung werden die spätere Form konturierende Schnitte in die getrocknete Masse hinein geführt, ohne den Papierträger zu beschädigen.
Das zwischen den nun erhaltenen einzeln dosierten Darreichungsformen verbliebene Material wird in einem Arbeitsgang durch mechanisches Abziehen entfernt.
Die Darreichungsformen werden auf dem Trägerpapier einzeln in einem im wesentlichen wasserdampfundurchlässigen Papier/-Aluminium/Ethylenvinylacetat-Verbundpackstoff eingesiegelt.
- Verwendung:: Instant-Zahnpasta

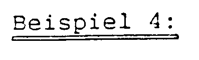

600 g acetylierte Stärke
440 g Calciumsulfat-Dihydrat
40 g Citronensäure
werden in eine mit zehn Mahlkörpern (Durchmesser ca. 4 cm) beschickte Porzellan-Kugelmühle eingewogen und in der verschlossenen Mühle eine Stunde bei 10 Upm trocken vorgemischt. Eine Suspension von
20 g Titandioxid in
550 g Wasser
wird zugesetzt und die Mischung eine weitere Stunde unter gleichen Bedingungen bewegt.

500 g Honig werden zugegeben; 2 Stunden wird weiter bei 10 Upm honogenisiert. Schließlich werden noch 16 ml Pfefferminzöl zugesetzt und die Rotation noch 18 Stunden lang fortgesetzt.
Die Weiterverarbeitung der so erhaltenen Masse erfolgt wie unter Beispiel 1 angegeben.

## Patentansprüche

1. Verfahren zur Herstellung einer folienförmigen, einzeln dosierten, in Wasser schnell zerfallenden Darreichungsform von Arzneimitteln, Süßwaren, anderen Lebenmitteln, Kosmetika zur oralen Anwendung oder Einnahme, wobei diese eine Grundmasse auf einem Träger enthält oder trägerfrei aus der Grundmasse besteht, gekennzeichnet durch folgende Schritte:
a) 20 bis 60 Gew.-% Filmbildner, 2 bis 40 Gew.-% Gelbildner, 0,1 bis 35 Gew.-% Wirkstoff und maximal 40 Gew.-% eines inerten Füllstoffes werden trocken innig vorgemischt;
b) die so erhaltene Vormischung aus Filmbildner, Gelbildner, Wirkstoff und Füllstoff wird unter Zusatz von bis zu 30 Gew.-%, bezogen auf die genannten Bestandteile, eines polaren Lösungsmittels unter Rühren in eine streichfähige Konsistenz gebracht, wobei fallweise zur Beschleunigung des Dispergierprozesses und Herbeiführung der gewünschten physikochemischen Beschaffenheit des Vorproduktes sowohl Hitze als auch Homogenisatoren und/oder Vakuum zur Anwendung gelangen;
c) die Masse des Vorproduktes wird auf ein Trennpapier oder eine Trennfolie aufgetragen, wobei die Schichtdicke der Masse 0,003 bis 4 Milimeter, vorzugsweise 20 bis 400 »m und insbesondere bevorzugt 70 bis 150 »m beträgt;
d) das polare Lösungsmittel wird nach Aufbringen der Schicht zumindest teilweise durch Einwirkung von Wärme und/oder Unterdruck entfernt.

2. Verfahren gemäß Anspruch 1, dadurch gekennzeichet, daß die streichfähige Masse ohne Lösungsmittelzusatz unter Verwendung eines bei Verarbeitungstemperatur schmelzenden Filmbildners hergestellt wird.

3. Verfahren gemäß Anspruch 1, dadurch gekennzeichnet, daß die Mischung der Komponenten gemäß a) unter Unterdruck vorgenommen wird.

4. Verfahren gemäß Anspruch 1, dadurch gekennzeichnet, daß die Mischung der Komponenten gemäß a) in einer Kugelmühle vorgenommen wird.

5. Verfahren gemäß Anspruch 1, dadurch gekennzeichnet, daß als Filmbildner im wesentlichen Gemische monomerer und oligomerer Zucker oder Zuckerderivate, Zuckeralkohole oder Polyethylenglykol verwendet werden.

6. Verfahren gemäß Anspruch 1, dadurch gekennzeichnet, daß als Gelbildner polymere Kohlehydrate oder deren Derivate, Gelatine, Carboxyvinylcopolymere, Polyvinylalkohol oder Gemische dieser Substanzen verwendet werden.

7. Verfahren gemäß Anspruch 1, dadurch gekennzeichnet, daß als Wirkstoff ein pharmakologisch aktiver Stoff verwendet wird.

8. Verfahren gemäß Anspruch 1, dadurch gekennzeichnet, daß als Füllstoff Calciumcarbonat, Calciumsulfat, ein Calciumphosphat, ein kristallin oder teilkristallin vorliegendes Kohlehydrat, Talkum, Titandioxid, Zinkoxid, Magnesiumstearat oder eine Mischung dieser Stoffe verwendet wird.

9. Verfahren gemäß Anspruch 1, dadurch gekennzeichnet, daß sie mindestens zwei wirkstoffhaltige Schichten enthält.

10. Verfahren gemäß Anspruch 1, dadurch gekennzeichnet, daß sie in vorproportionierter Form einzeln dosiert vorliegt.

## Claims

1. Process for the production of a sheet-like, individually dosed administration form of drugs, confectionary, other foodstuffs, cosmetics and the like for oral application or intake, which administration form rapidly disintegrates in water, whereby said administration form contains a base mass on a carrier or consists of the base mass without a carrier, characterized by the following steps:
a) 20 to 60%-wt. of film forming agent(s), 2 to 40%-wt. of gel-forming agent(s), 0.1 to 35%-wt. of active substance and a maximum of 40%-wt. of an inert filling agent are intimately premixed in dry condition;
b) the initial mixture thus obtained, comprising film former, gel former, active substance and filling agent, is given a spreadable consistency by adding up to 30%-wt. of a polar solvent, relative to the above-mentioned components, while stirring, whereby, in order to accelerate the dispersion process and to produce the desired physicochemical properties of the initial product, from case to case both heat and homogenizers and/or vacuum are employed;
c) the mass of the initial product is applied to a release paper or a release film, the thickness of the mass amounting to 0.003 to 4 mm, preferably 20 to 400 »m, and particularly preferred 70 to 150 »m.
d) the polar solvent is, after application of the layer, at least partially removed by the action of heat and/or low pressure.

2. The process according to claim 1, characterized in that the spreadable mass is manufactured without the addition of a solvent but employing a film former melting at the process temperature.

3. The process according to claim 1, characterized in that the mixing of the components according to a) is carried out under partial vacuum.

4. The process according to claim 1, characterized in that the mixing of the components according to a) is carried out in a ball mill.

5. The process according to claim 1, characterized in that as film formers there are used, substantially, mixtures of monomeric or oligomeric sugars or sugar derivatives of sugar alcohols or polyethylene glycol.

6. The process according to claim 1, characterized in that as gel formers there are used polymeric carbohydrates or the derivatives thereof, gelatin, carboxyvinyl copolymers, polyvinyl alcohol, or mixtures of said substances.

7. The process according to claim 1, characterized in that as active substance a pharmacologically active substance is used.

8. The process according to claim 1, characterized in that as filler calcium carbonate, calcium sulfate, a calcium phosphate, a carbohydrate present in a crystalline or partially crystalline form, talc, titanium dioxide, zinc oxide, magnesium stearate, or a mixture of these substances is used.

9. The process according to claim 1, characterized in that the administration form comprises at least two active substance-containing layers.

10. The process according to claim 1, characterized in that the administration form is present in portions which are individually dosed.

## Revendications

1. Procédé de préparation d'une forme d'administration de médicaments, de produits de confiserie, d'autres aliments, de produits cosmétiques, pour l'ingestion ou l'utilisation orale, sous forme de feuilles, individuellement dosée, se décomposant rapidement dans l'eau, où cette forme d'administration contient une masse de base sur un support, ou se compose de la masse de base sans support, caractérisé en ce qu'il comprend les étapes suivantes consistant à :
a) prémélanger intimement à sec 20 à 60% en poids d'un agent filmogène, 2 à 40% en poids d'un agent géligène, 0,1 à 35% en poids de principe actif et, au maximum, 40% en poids d'une charge inerte,
b) amener le prémélange ainsi obtenu d'agent filmogène, d'agent géligène, de principe actif et de charge à une consistance permettant l'étalement en recourant à l'addition de jusqu'à 30% en poids, par rapport aux constituants précités, d'un solvant polaire, sous agitation, où le cas échéant, pour accélérer le processus de dispersion et pour conférer la compatibilité physicochimique souhaitée au produit préalable, on utilise aussi bien la chaleur qu'également des homogénéisateurs et/ou le vide,
c) appliquer la masse du produit préalable sur un papier séparateur ou une feuille séparatrice, où l'épaisseur de couche de la masse atteint 0,003 à 4 mm, de préférence 20 à 400 »m et, de manière particulièrement avantageuse, 70 à 150 »m,
d) éliminer le solvant polaire après l'application de la couche, au moins partiellement sous l'influence de la chaleur et/ou d'une pression inférieure à la pression atmosphérique.

2. Procédé suivant la revendication 1, caractérisé en ce que la masse étalable est préparée sans addition de solvant en recourant à l'emploi d'un agent filmogène fondant à la température de mise en oeuvre.

3. Procédé suivant la revendication 1, caractérisé en ce que l'on entreprend le mélange des composants selon a) sous une pression inférieure à la pression atmosphérique.

4. Procédé suivant la revendication 1, caractérisé en ce que l'on entreprend le mélange des composants selon a) dans un broyeur à billes.

5. Procédé suivant la revendication 1, caractérisé en ce que l'on utilise, à titre d'agent filmogène, essentiellement des mélanges de sucres monomériques et oligomériques ou de dérivés de sucre, d'alcools sucres ou de polyéthylèneglycol.

6. Procédé suivant la revendication 1, caractérisé en ce que l'on utilise, à titre de polymères géligènes, des hydrates de carbone ou leurs dérivés, des gélatines, des copolymères carboxyvinyliques, le poly(alcool vinylique), ou des mélanges de ces substances.

7. Procédé suivant la revendication 1, caractérisé en ce que l'on utilise une substance pharmacologiquement active à titre de principe actif.

8. Procédé suivant la revendication 1, caractérisé en ce que l'on utilise, à titre de charge, du carbonate de calcium, du sulfate de calcium, un phosphate de calcium, un hydrate de carbone se présentant sous forme cristalline ou partiellement cristalline, du talc, du dioxyde de titane, de l'oxyde de zinc, du stéarate de magnésium, ou un mélange de ces substances.

9. Procédé suivant la revendication 1, caractérisé en ce que la forme d'administration contient au moins deux couches contenant du principe actif.

10. Procédé suivant la revendication 1, caractérisé en ce que la forme d'administration se présente en dosage individuel sous forme proportionnée au préalable.
